(19) Europäisches Patentamt
European Patent Office
Office européen des brevets

(11) Publication number: **0 048 039**
**A1**

(12) # EUROPEAN PATENT APPLICATION

(21) Application number: 81200594.0

(22) Date of filing: 01.06.81

(51) Int. Cl.$^3$: **A 01 N 43/10**
A 01 N 43/08, C 07 D 333/24
C 07 D 307/54, C 07 D 207/32

(30) Priority: 20.06.80 GB 8020304

(43) Date of publication of application:
24.03.82 Bulletin 82/12

(84) Designated Contracting States:
BE CH DE FR GB IT LI NL

(71) Applicant: SHELL INTERNATIONALE RESEARCH MAATSCHAPPIJ B.V.
Carel van Bylandtlaan 30
NL-2596 HR DEN HAAG(NL)

(72) Inventor: Haddock, Ernest
187 Queensborough Road Sheerness
Sheppey Kent(GB)

(72) Inventor: Hopwood, William John
24 Blandford Gardens
Sittingbourne Kent(GB)

(74) Representative: Rogers, Roy Charles et al,
4 York Road
London SE1 7NA(GB)

(54) **Compounds that contain a heterocycle, process for their preparation, compositions containing them for regulating the growth of plants, and their use.**

(57) A plant growth regulating composition which comprises a carrier and, as active ingredient, a compound of the general formula I or, where possible, an acid addition salt thereof:

$$\text{Het} - \overset{\overset{\large X}{|}}{\underset{\underset{\large CN}{|}}{C}} - \overset{\overset{\large Y}{|}}{N} - \text{Ar} \qquad (I)$$

in which Het represents an optionally substituted 5-membered heterocyclic ring containing one hetero atom; Ar represents an optionally substituted phenyl group; and either X and Y together represent a bond, or X represents a hydrogen atom or an alkyl group and Y represents a hydrogen atom or an acyl group derived from a carboxylic acid; and a method of regulating the growth of plants.

EP 0 048 039 A1

Croydon Printing Company Ltd.

Plant growth regulating compositions, a method for regulating the growth of plants using the compositions, novel heterocycle-containing aniline compounds and a process for their preparation

This invention relates to a composition for and to a method of regulating the growth of plants and to compounds for use in such a composition or method.

In the past, the vast majority of agricultural chemical research work was directed towards the development of chemical fertilizers and the development of chemicals to combat pests, diseases and weeds which reduce the yield of crops. The belief was that by adequately feeding the plant and by optimizing its environment, the yields of the crop could be maximized.

In recent years it has become clear that it is possible to affect the growth of plants in a much more fundamental way, that is by the application of chemicals which affect the biochemical processes occuring in the plant. Much research is now being directed towards the development of chemical plant growth regulants which are capable of altering the biochemistry of plants in such a way that useful effects are achieved. One important field of research is into the stimulation of crops to produce higher yields. It would for example be most useful to be able to increase the yield of soyabeans, a valuable source of protein, by means of a chemical treatment.

It has now been found that certain heterocycle-containing aniline compounds have useful plant growth regulating activity. "Chemistry and Industry", 3rd February 1968, p.155, discloses that certain N(1-heterocyclyl-1-cyanoethyl)anilines may be

prepared by reaction of a ketone with aniline and cyanide ion in the presence of acetic acid. There is no suggestion, however, that these compounds have any use in agriculture.

The invention provides a plant growth regulating composition which comprises a carrier and, as active ingredient, a compound of the general formula I or, where possible, an acid addition salt thereof:

$$\mathrm{Het} - \overset{\overset{\textstyle X}{|}}{\underset{\underset{\textstyle CN}{|}}{C}} - \overset{\overset{\textstyle Y}{|}}{N} - \mathrm{Ar} \qquad (I)$$

in which Het represents an optionally substituted 5-membered heterocyclic ring containing one hetero atom; Ar represents an optionally substituted phenyl group; and either X and Y together represent a bond, or X represents a hydrogen atom or an alkyl group and Y represents a hydrogen atom or an acyl group derived from a carboxylic acid.

The ring Het may be saturated or unsaturated and may for example be unsubstituted or substituted by one or more, preferably one or two, of the same or different substitutents selected from halogen atoms, alkyl groups, for example methyl groups, and nitro groups. When Het represents a pyrrolyl group, this may carry an N-oxide substituent. Preferably the ring Het is unsubstituted, for example, it may be a 2- or 3- furyl, thienyl or pyrrolyl group or one of the corresponding tetrahydro groups. An especially preferred group Het is a 2-thienyl group.

The group Ar is preferably unsubstituted or substituted by one or more, especially one or two, of the same or different substituents selected from halogen atoms and alkyl, haloalkyl, alkoxy, haloalkoxy, nitro, cyano, alkanoyl, carboxy and alkoxycarbonyl groups. Especially preferred substituents are halogen atoms and methyl, methoxy and trifluoromethyl groups. Most preferably, the group Ar carries one or two substituents, and most preferably these substituents are halogen atoms.

In the substituents mentioned above for the groups Het and Ar, unless otherwise stated, any halogen atom present is preferably a fluorine, bromine or chlorine atom, and any alkyl

moiety preferably has up to 4, especially 1 or 2, carbon atoms.

If X represents an alkyl group, this preferably has up to 4 carbon atoms, for example X may represent a methyl group. If Y represents an acyl group derived from a carboxylic acid, this group is preferably an optionally substituted alkanoyl or benzoyl group. An alkanoyl group may for example be substituted by one or more of the same or different substituents selected from halogen atoms and alkoxy, phenyl, phenoxy, carboxy and alkoxycarbonyl groups, but preferably an alkanoyl group Y is unsubstituted. Preferably an alkanoyl group Y has up to 4 carbon atoms, for example Y may represent an acetyl group. If Y represents an optionally substituted benzoyl group, the preferred substituents are as given above for the group Ar.

Preferably X and Y together represent a bond, or X and Y both represent hydrogen atoms.

When Y represents a hydrogen atom, the compounds of the general formula I can form acid addition salts, which may be present as active ingredient in the compositions according to the invention. Suitable acids include organic acids, for example tartaric or citric acid, and inorganic acids, for example hydrochloric acid.

Most of the compounds of the general formula I are novel, and the invention therefore also provides these novel compounds *per se*. The novel compounds are those of the general formula I and acid addition salts thereof as defined above, provided that if Y represents a hydrogen atom and either X represents a methyl group or Het represents an unsubstituted 2-furyl ring and X represents a hydrogen atom, then Ar must represent a substituted phenyl group.

The invention also provides a process for the preparation of a novel compound of the general formula I, which comprises reacting a compound of the general formula

$$\text{Het} - \overset{\overset{\displaystyle X}{|}}{C} = N - \text{Ar} \qquad \text{(II)}$$

with a compound of the general formula Y.CN; and in the event of a compound of the general formula

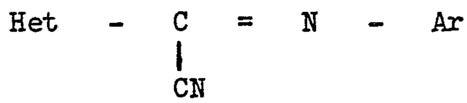
(Ia)

being required, reacting a resulting compound of the general formula

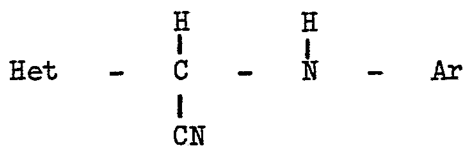
(Ib)

with an oxidizing agent; and in the event of a compound of the general formula

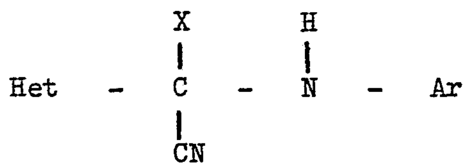
(Ic)

being produced and a compound of the general formula

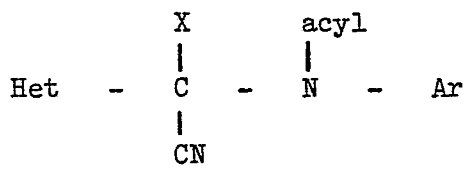
(Id)

being required, reacting said compound of the formula (Ic) with a suitable acylating agent.

Hydrogen cyanide may be added to the compound of the general formula II as such, in the form of a solution, a gas or a liquid, or it may be generated in situ, for example by the action of an acid on an alkali metal cyanide. The acid may be a mineral acid, such as hydrochloric acid, or an organic acid, such as acetic acid.

Preferred acyl cyanides are optionally substituted alkanoyl or benzoyl cyanides, for example acetyl or benzoyl cyanide.

The reaction of the compound of the general formula II with the compound of formula Y.CN may be carried out over a wide temperature range, for example within the range of from 0 to 150°C. It is conveniently carried out at the reflux temperature

of the reaction mixture, or at room temperature. The reaction is preferably carried out in the presence of a suitable solvent, for example an ether, an alcohol, a hydrocarbon or a chlorinated hydrocarbon. The molar ratio of the reactants may vary over a wide range. An excess of the reactant Y.CN, for example up to a 5 fold excess, may be used, but preferably approximately stoichiometric quantities are used.

To produce a compound of formula I in which X and Y together represent a bond, a compound of formula Ib is prepared, and then reacted with an oxidizing agent, for example manganese dioxide. Any suitable solvent, for example benzene, may be used if desired, and the reaction temperature is preferably in the range of from 0 to 100°C. Conveniently the reaction may be carried out at room temperature.

Rather than using as reactant a compound of formula Y.CN when Y is an acyl group, it may be preferred to prepare a compound of formula Id by acylation of a resulting compound of formula Ic. Any suitable acylating agent, for example a carboxylic acid, an acyl halide or an acyl anhydride, may be used. Preferably, an acyl chloride is used, and the reaction is then preferably carried out in the presence of an acid binding agent, which may be an organic or inorganic base. Organic amines, for example triethylamine, are especially suitable. The reaction is preferably carried out in the presence of an inert solvent, for example a hydrocarbon such as benzene, at a temperature in the range of from 50 to 150°C. The reaction is conveniently carried out under reflux.

Compounds of the general formula II are Schiff bases and can be prepared by methods analogous to known methods, for example by reacting an aldehyde or ketone of formula Het.CO.X, with an aniline of formula $ArNH_2$.

The compounds of the general formula I have plant growth regulating properties and the invention provides a method of regulating the growth of a plant at a locus, which comprises applying to the locus a compound or a composition according to

the invention. An especially useful plant growth regulating effect is the increase in the number of pods and/or seed weight (yield) in soyabean plants, and the locus to be treated is therefore preferably a soyabean crop. The effect and efficacity of the treatment will in general depend on the growth stage of the crop treated. To promote increased numbers of pods in soyabean plants at time of harvest, the treatment is suitably carried out during the period from the time at which the flowers have differentiated up to the time that the pods have begun to fill. In this same respect the most significant increases in crop yield seem to occur when the soyabean crop is treated at about the midway stage of the filling of the pods.

Preferably the growth of the plants is regulated by the application of the active compound at a dosage in the range of from 0.05 to 4 kg/ha, preferably 0.25 to 1.5 kg/ha.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating agricultural compositions may be used. A composition according to the invention preferably contains from 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen;

waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 and 75% w of active ingredient and usually contain, in addition to solid inert carrier, 3-10% w of a dispersing agent and, where necessary, 0-10% w of stabiliser(s) and/or other additives such as penetrants or stickers. Dusts are usually formulated as a dust concentrate having a similar compositions to that of a wettable powder but without a dispersant, and are diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain ½-25% w active ingredient and 0-10% w of additives such as stabilisers, slow release modifiers and binding agents. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil

or of the oil-in-water type, and may have a thick 'mayonnaise'-like consistency.

The compositions of the invention may also contain other ingredients, for example, other compounds possessing plant growth regulating, insecticidal or fungicidal properties.

The following Examples illustrate the invention.

Example 1 : Preparation of 2-thienyl-N-3,4-dichlorophenylamino-acetonitrile

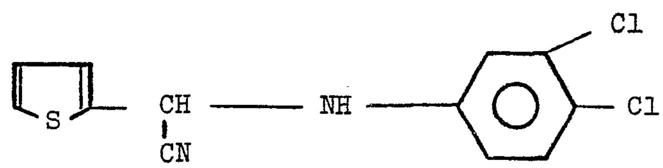

2-Thiophenecarboxaldehyde (5.6g), was mixed with 3,4-dichloroaniline (8.1g) and heated at 100°C for 5 hours. In order to remove water, 200 ml chloroform were then added and evaporated off. The resultant semi-solid was recrystallized from ethanol to give 6.6g of 2-thienylcarboxaldehyde-3,4-dichlorophenylimine, as a white solid, melting point 82-83°C. This corresponded to a yield of 52%.

5.1g of this solid were mixed with 1.3g potassium cyanide, 1.3g acetic acid and 50ml ethanol, and refluxed for 5 hours, when all the solid had disappeared. The solvent was then evaporated off and the residue was partitioned between water and ethyl acetate. The organic layer was separated off, dried over sodium sulphate and evaporated. The residue was recrystallized from petrol (boiling point 60-80°C) to give 4.6g of the desired product, melting point 103-105°C. This corresponds to a yield in this step of 81.3%.

Elemental Analysis

| | C | H | N |
|---|---|---|---|
| Calculated for $C_{12}H_8N_2Cl_2S$ | 50.9 | 2.8 | 9.9 |
| Found | 50.9 | 2.9 | 9.6 |

Example 2 Preparation of 2-thienylcarboxaldehyde-α-cyano-3,4-dichlorophenylimine

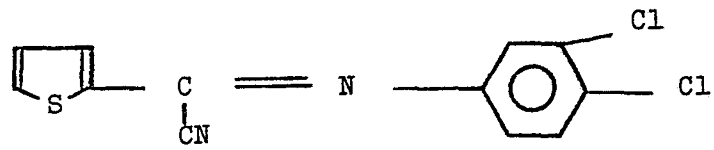

5g of the compound of Example 1 were dissolved in benzene (100 ml), manganese dioxide (5g) was added, and the mixture was stirred at room temperature for 15 hours. It was then filtered and evaporated down, and the solid yellow residue was recrystalized from benzene. 4.9g of the desired product, melting point 106-108°C, were obtained, corresponding to a yield of 98.9%

Elemental Analysis

| | C | H | N |
|---|---|---|---|
| Calculated for $C_{12}H_6N_2Cl_2S$ | 51.25 | 2.14 | 9.96 |
| Found | 51.4 | 2.1 | 10.0 |

Example 3 Preparation of 2-thienyl-N-chloromethylcarbonyl-N-3,4-dichlorophenylamino-acetonitrile

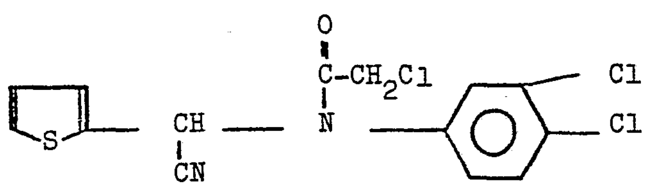

2-Thienyl-N-3,4-dichlorophenylamino-acetonitrile (5.6g) prepared as described in Example 1 was dissolved in xylene (50ml) containing chloroacetyl chloride (10ml). The reaction mixture was then refluxed until the evolution of hydrogen chloride gas ceased. Subsequently the solvent was evaporated and the residual gum was triturated with petrol (boiling point 60-80°C) to afford the desired product (5.1g) as a white solid melting at 100-102°C. This corresponds to a yield of 98%.

Elemental Analysis

| | C | H | N |
|---|---|---|---|
| Calculated for $C_{14}H_9N_2Cl_3S$ | 46.7 | 2.5 | 7.8 |
| Found | 46.7 | 2.5 | 7.8 |

Examples 4-21

By methods analogous to those described in Examples 1 and 2, the compounds of formula I given in Table I were prepared.

Table I

| Example No. | In the general formula I | | | | Melting Point | Elemental Analysis | | | | | |
|---|---|---|---|---|---|---|---|---|---|---|---|
| | Het | Ar | X | Y | °C | Calculated | | | Found | | |
| | | | | | | C | H | N | C | H | N |
| 4 | 2-thienyl | 2,4-dichloro-phenyl | H | H | 134 - 136 | 50.9 | 2.8 | 9.9 | 50.9 | 2.9 | 9.9 |
| 5 | 2-furyl | 2,4-dichloro-phenyl | H | H | 52 - 54 | 53.9 | 3.0 | 10.5 | 53.7 | 3.0 | 10.4 |
| 6 | 2-furyl | 3,4-dichloro-phenyl | H | H | 64 - 66 | 53.9 | 3.0 | 10.5 | 53.7 | 3.1 | 10.5 |
| 7 | 2-furyl | 2,4-dichloro-phenyl | bond | | 113 - 115 | 54.3 | 2.3 | 10.6 | 53.8 | 2.3 | 10.2 |
| 8 | 2-furyl | 3,4-dichloro-phenyl | bond | | 44 - 46 | 54.3 | 2.3 | 10.6 | 54.3 | 2.3 | 9.9 |
| 9 | 2-thienyl | 2,4-dichloro-phenyl | bond | | 60 - 62 | 51.3 | 2.1 | 10.0 | 51.3 | 2.1 | 10.1 |

Table I continued....

| Example No. | Het | Ar | X | Y | Melting Point °C | Calculated C | Calculated H | Calculated N | Found C | Found H | Found N |
|---|---|---|---|---|---|---|---|---|---|---|---|
| 10 | 5-methyl-2-thienyl | 3,4-dichloro-phenyl | H | H | - | 55.5 | 3.6 | 9.9 | 55.3 | 3.7 | 9.4 |
| 11 | 2-thienyl | 3,4-dichloro-phenyl | H | $-\overset{O}{\overset{\Vert}{C}}-C(CH_3)_3$ | 84-86 | 55.6 | 4.4 | 7.6 | 55.2 | 4.4 | 7.5 |
| 12 | 2-thienyl | phenyl | H | H | 68-70 | 64.7 | 4.9 | 12.6 | 65.2 | 4.2 | 12.5 |
| 13 | 2-thienyl | phenyl | bond | | 92-94 | 67.9 | 3.8 | 13.2 | 67.1 | 3.8 | 13.0 |
| 14 | 3-methyl-2-thienyl | 3,4-dichloro-phenyl | H | H | - | 53.3 | 3.3 | 5.2 | 53.5 | 3.5 | 5.1 |
| 15 | 3-methyl-2-thienyl | 3,4-dichloro-phenyl | bond | | 87-89 | 53.0 | 2.7 | 9.5 | 53.6 | 2.7 | 9.6 |
| 16 | 2-thienyl | 4-bromophenyl | bond | | 138-139 | 49.5 | 2.4 | 9.6 | 49.5 | 2.3 | 9.6 |
| 17 | 2-thienyl | 4-bromophenyl | H | H | 89-90 | 49.1 | 3.1 | 9.5 | 49.2 | 3.1 | 9.3 |
| 18 | 2-thienyl | 3-trifluoromethyl-phenyl | H | H | 72-75 | 55.3 | 3.2 | 9.9 | 55.6 | 3.3 | 9.9 |
| 19 | 2-thienyl | 3-trifluoromethyl-phenyl | bond | | 65-66 | 55.7 | 2.5 | 10.0 | 55.8 | 2.5 | 10.0 |
| 20 | 2-thienyl | 4-chlorophenyl | H | H | 92-94 | 57.9 | 3.6 | 11.3 | 58.3 | 3.8 | 11.0 |
| 21 | 2-thienyl | 4-chlorophenyl | bond | | 103-104 | 58.4 | 2.8 | 11.3 | 58.0 | 2.6 | 11.4 |

Example 22 Demonstration of soyabean growth regulation

The plants used in the evaluation of the compounds were determinate soyabean cultivars.

Liquid formulations of the test compounds were applied to plants at an early stage of flower development. The formulations used consisted of 60% water, 40% acetone which contained 0.4% TRITON X155 (Trade Mark), and amounts of the test compound to give spray applications over a range of dosages as given in Table II.

In order to induce nodulation, the soyabean seeds were inoculated with a commercial strain of Rhizobium ("Nitrogerm", Root Nodule Pty., Ltd., Australia) prior to sowing in a loam: grit (5:1) mixture in 5" diameter pots. Plants were maintained at 21-25°C under 14 hour day length and watered by sub-irrigation. Treatments were as foliar applications to three plants for each dose of the test material, applied in a volume equivalent to 632 litres per hectare. After treatment the plants were set out in a randomised block design. After four weeks the total pod number per plant was recorded. Results were derandomised and mean values calculated and expressed as a percentage of the untreated controls. The pod numbers thus obtained are given in Table II.

TABLE II

| Compound of Example No. | Soyabean Pod Numbers % of Untreated Control | | | | | |
|---|---|---|---|---|---|---|
| Dosage (Kg/ha) | 0.3 | 0.5 | 1 | 2 | 3 | 5 |
| 1 | - | 143 | - | 131 | - | 131 |
| 2 | - | 119 | 138 | 100 | - | - |
| 3 | 91 | - | - | - | 112 | - |
| 4 | - | 88 | 106 | 100 | - | - |
| 5 | - | 119 | 113 | 113 | - | - |
| 6 | - | 130 | 120 | 130 | - | - |
| 7 | - | 156 | 125 | 119 | - | - |
| 10 | 102 | - | - | | 110 | - |
| 11 | 108 | - | - | - | 73 | - |
| 12 | 96 | - | - | - | 105 | - |
| 13 | 99 | - | - | - | 92 | - |
| 14 | 105 | - | - | - | 114 | |
| 15 | 93 | - | - | - | 121 | - |
| 16 | 101 | - | - | - | 105 | - |
| 17 | 107 | - | - | - | 117 | - |
| 18 | 105 | - | - | - | 106 | - |
| 19 | 101 | - | - | - | 102 | - |
| 20 | 89 | - | - | - | 115 | - |
| 21 | 107 | - | - | - | 105 | - |

CLAIMS

1. A plant growth regulating composition which comprises a carrier and, as active ingredient, a compound of the general formula I or, where possible, an acid addition salt thereof:

$$\begin{array}{ccccccc} & & X & & Y & & \\ & & | & & | & & \\ Het & - & C & - & N & - & Ar \\ & & | & & & & \\ & & CN & & & & \end{array} \qquad (I)$$

in which Het represents an optionally substituted 5-membered heterocyclic ring containing one hetero atom; Ar represents an optionally substituted phenyl group; and either X and Y together represent a bond, or X represents a hydrogen atom or an alkyl group and Y represents a hydrogen atom or an acyl group derived from a carboxylic acid.

2. A composition as claimed in claim 1, in which the group Het is unsubstituted or substituted by one or more of the same or different substituents selected from halogen atoms, alkyl groups and nitro groups.

3. A composition as claimed in claim 2, in which Het represents an unsubstituted 2- or 3-furyl, thienyl or pyrrolyl group or one of the corresponding tetrahydro groups.

4. A composition as claimed in claim 3, in which Het represents a 2-thienyl group.

5. A composition as claimed in any one of claims 1 to 4, in which Ar represents a phenyl group which is unsubstituted or substituted by one or more of the same or different substituents selected from halogen atoms and alkyl, haloalkyl,

alkoxy, haloalkoxy, nitro, cyano, alkanoyl, carboxy and alkoxycarbonyl groups.

6. A composition as claimed in claim 5, in which Ar represents a phenyl group which is unsubstituted or substituted by one or two of the same or different substituents selected from halogen atoms and methyl, methoxy and trifluoromethyl groups.

7. A composition as claimed in claim 6, in which Ar represents a dihalophenyl group.

8. A composition as claimed in any one of claims 1 to 7, in which either X and Y together represents a bond, or X represents a hydrogen atom or an alkyl group having up to 4 carbon atoms and Y represents a hydrogen atom, an alkanoyl group having up to 4 carbon atoms in the alkyl moiety, or a group of formula $Ar^1CO-$ where $Ar^1$ has the meaning given for the group Ar in any one of claims 5 to 7.

9. A composition as claimed in claim 8, in which either X and Y together represent a bond, or X and Y both represent hydrogen atoms.

10. A composition as claimed in claim 1, in which the compound of formula I is any one of those named in Examples 1 to 21 herein.

11. A composition as claimed in any one of claims 1 to 10, which comprises at least two carriers, at least one of which is a surface-active agent.

12. A compound of the general formula I or where possible an acid addition salt thereof, as defined in any one of claims 1 to 9, provided that if Y represents a hydrogen atom and either X represents a methyl group or Het represents an unsubstituted 2-furyl ring and X represents a hydrogen atom, then Ar must represent a substituted phenyl group.

13. A compound as claimed in claim 12 and named in any one of Examples 1 to 21 herein.

14. A process for the preparation of a compound as claimed in claim 12, which comprises reacting a compound of the general formula:

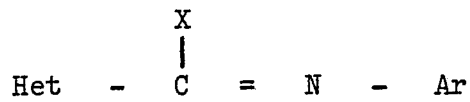 (II)

with a compound of the general formula Y.CN; and in the event of a compound of the general formula

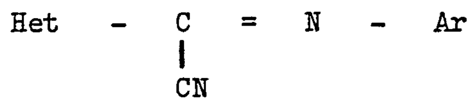 (Ia)

being required, reacting a resulting compound of the general formula

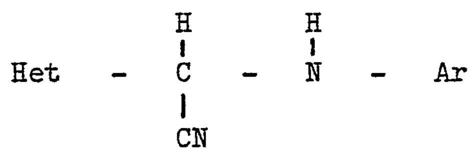 (Ib)

with an oxidizing agent; and in the event of a compound of the general formula

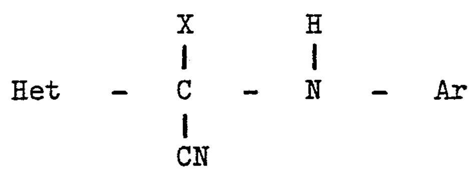 (Ic)

being produced and a compound of the general formula

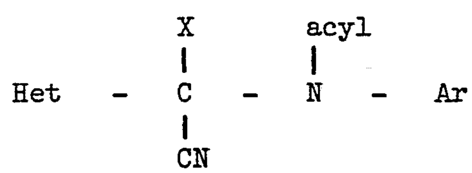 (Id)

being required, reacting said compound of the formula (Ic) with a suitable acylating agent.

15. A process as claimed in claim 14, in which the compound of formula Y.CN is hydrogen cyanide which is generated <u>in situ</u> by the action of an acid on an alkali metal cyanide.

16. A process as claimed in either claim 14 or claim 15, in which the compound of formula Y.CN is reacted with the compound of the general formula II at a temperature in the range of from 0 to 150°C.

17. A process as claimed in any one of claims 14 to 16, which includes the step of reacting a resulting compound of the

general formula Ib with an oxidizing agent, the oxidizing agent being manganese dioxide.

18. A process as claimed in any one of claims 14 to 16, which includes the step of reacting a resulting compound of the general formula Ic with an acyl chloride.

19. A compound as claimed in claim 12, whenever prepared by a process as claimed in any one of claims 14 to 18.

20. A method of regulating the growth of a plant at a locus, which comprises applying to the locus a composition as claimed in any one of claims 1 to 11 or a compound as claimed in any one of claims 12, 13 and 19.

21. A method as claimed in claim 20, in which the locus is a soyabean crop.

0048039

European Patent Office

# EUROPEAN SEARCH REPORT

Application number
EP 81 20 0594

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| | CHEMICAL ABSTRACTS, vol. 85, no.7, August 16, 1976 page 536, abstract 62772s Columbus, Ohio, USA J.N. ACHARYA et al.."Preparation of nitriles and amides of benzocaine as potential drugs" & J. INDIAN. CHEM. SOC. 1976, 53(3), 307-8 * Abstract * | 1-21 | A 01 N 43/10<br>43/08<br>C 07 D 333/24<br>307/54<br>207/32 |
| | -- | | |
| | DE - A - 2 221 771 (BAYER) * Pages 1,2,24 and claims pages 25-26 * | 1-21 | |
| | ---- | | |

TECHNICAL FIELDS SEARCHED (Int. Cl.3)

A 01 N 43/08
43/10
C 07 D 207/32
307/54
333/24

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

x The present search report has been drawn up for all claims

| Place of search | Date of completion of the search | Examiner |
|---|---|---|
| The Hague | 25-09-1981 | MAISONNEUVE |

EPO Form 1503.1 06.78